# NEUE EUROPÄISCHE PATENTSCHRIFT

(11) **EP 2 343 986 B2**
(45) Veröffentlichungstag und Bekanntmachung des Hinweises auf die Entscheidung über den Einspruch: **21.04.2021**
(45) Hinweis auf die Patenterteilung: 06.01.2016
(21) Anmeldenummer: 09783652.2
(22) Anmeldetag: 01.10.2009
(51) Int. Cl.: A23L 2/38, A23D 7/01, A61K 8/31, A61K 8/67, A61K 8/73, A61Q 19/00, A23L 1/30, A23L 2/52, A23K 1/16, A61K 8/34, A61K 8/06, A23L 1/302, A23D 7/005

(54) **GEBRAUCHFERTIGE, STABILE EMULSION**
READY-TO-USE, STABLE EMULSION
ÉMULSION STABLE PRÊTE À L'EMPLOI

(30) Priorität: 07.10.2008 EP 08165989
(43) Veröffentlichungstag der Anmeldung: 20.07.2011
(73) Patentinhaber: BASF SE, 67056 Ludwigshafen am Rhein (DE)
(72) Erfinder: KÖPSEL, Christian, 69469 Weinheim (DE)
(74) Vertreter: BASF IP Association
(86) Internationale Anmeldenummer: PCT/EP2009/062768
(87) Internationale Veröffentlichungsnummer: WO 2010/040683

(56) Entgegenhaltungen:
- EP-A- 0 551 638
- EP-A- 1 875 814
- EP-A- 1 927 287
- EP-A1- 1 782 803
- EP-A1- 1 875 814
- EP-A2- 0 835 613
- EP-A2- 0 845 503
- WO-A-2007/009601
- WO-A-2008/087140
- WO-A1-2005/075066
- WO-A1-2007/003543
- WO-A1-2007/009601
- WO-A1-2010/112406
- WO-A2-2008/087140
- Belitz, H.-D., Grosch, W.: "Lehrbuch der Lebensmittelchemie. 3 Auflage.", , 1987, pages 1-6, Springer Verlag, Berlin
- Research Disclosure 452 072
- Product Data Sheet Purity Gum, 27.07.2000

## Beschreibung

Die vorliegende Erfindung betrifft eine gebrauchsfertige, stabile Emulsion von fettlöslichen Vitaminen oder Carotinoiden sowie ihre Verwendung als Zusatz zu Tierfuttermitteln, Lebensmitteln und Nahrungsergänzungsmitteln sowie kosmetischen und pharmazeutischen Mitteln.

Die fettlöslichen Vitamine A, D, E oder K sowie ihre Derivate und die Carotinoide wie beispielsweise Canthaxanthin, Astaxanthin, Zeaxanthin, Lutein, Lycopin oder β-Carotin werden in der Futtermittel- und Lebensmittelindustrie, der Kosmetikindustrie und der pharmazeutischen Industrie häufig nicht direkt als Reinstoffe eingesetzt. Vielmehr werden Formulierungen dieser fettlöslichen Substanzen verwendet, wodurch es ermöglicht wird, die fettlöslichen Substanzen in fein verteilter Form homogen in einem wasserhaltigen Medium zu dispergieren. Kommerziell werden je nach Anwendungsgebiet sowohl feste als auch flüssige Formulierungen dieser fettlöslichen Substanzen eingesetzt.

Während die Vitamine A, D oder K in der Regel auf Grund ihrer physiologischen Wirkung im lebenden Organismus eingesetzt werden, wird Vitamin E häufig auch als Antioxidationsmittel in vitro in verschiedenen Anwendungen benutzt. Die Carotinoide, welche ebenfalls über eine antioxidative Wirkung verfügen, werden in vielen Anwendungen als Farbstoffe eingesetzt und können dabei gleichzeitig eine physiologische Funktion erfüllen. β-Carotin ist beispielsweise Farbstoff und Provitamin A.

In der Getränkeindustrie werden Zusatzstoffe in der Regel in Form von flüssigen Konzentraten den Getränken zugesetzt. Im Falle von wasserlöslichen, pulverförmigen Formulierungen werden üblicherweise im Produktionsprozess zunächst wässrige Dispersionen aus diesen Pulvern hergestellt. Im Falle von flüssigen Formulierungen, wie beispielsweise Emulsionen, entfällt dieser Verfahrensschritt.

In EP 0 239 086 werden Emulsionen eines in Öl gelösten Carotinoids beschrieben, wobei zur Stabilisierung der Öltröpfchen eine Mischung aus einem Ester einer langkettigen Fettsäure mit Ascorbinsäure und einem kaltwasserlöslichen Stärkeprodukt, wie beispielsweise Stärkeoctenylsuccinat, eingesetzt wird. Die Carotinoidkonzentration liegt in diesen Emulsionen zwischen 0,1 und 2 %.

In EP 0 551 638 werden stabile flüssige Emulsionen von fettlöslichen Vitaminen oder Carotinoiden hergestellt, wobei die äußere Phase Glycerin oder ein Glycerin-WasserGemisch ist und als Emulgator und Stabilisator ein Ester der Ascorbinsäure mit langkettigen Fettsäuren eingesetzt wird. Im Falle von β-Carotin zeichnen sich die Präparate durch einen strahlenden Gelbton aus.

Die aus dem Stand der Technik bekannten Emulsionen von fettlöslichen Wirkstoffen, die den Emulgator Ascorbylpalmitat enthalten, sind für die Anwendung in mineralienreichen Sportlergetränken oder bei Verwendung von Trinkwasser mit einem hohen Gehalt an Calcium- oder Magnesiumionen noch nicht zufriedenstellend, da in den Getränkeflaschen eine Ringbildung beobachtet wird, was auf eine Instabilität der eingesetzten Emulsion hinweist.

Der vorliegenden Erfindung lag die Aufgabe zugrunde, eine gebrauchsfertige, stabile Emulsion zur Verfügung zu stellen, die sowohl eine gute Lagerstabilität im Hinblick auf mikrobiologischen Befall aufweist, als auch thermisch unempfindlich ist und insbesondere eine verbesserte Stabilität im Einsatz in Calcium- oder Magnesium-haltigen Getränken oder bei Verwendung von Trinkwasser mit einem hohen Gehalt an Calcium-oder Magnesiumionen zeigt.

Diese Aufgabe wird durch eine gebrauchsfertige, stabile Emulsion umfassend
a) eine dispergierte Ölphase, die ein fettlösliches Vitamin oder ein Carotinoid umfasst,
   und
b) eine wässrige Phase, die 30 bis 55 Gew.-% Glycerin und eine chemisch modifizierte Stärke als wasserlösliches Schutzkolloid umfasst,
worin der Gehalt an dem fettlöslichen Vitamin oder an dem Carotinoid mindestens 2 Gew.-% beträgt, und der Gehalt einer weiteren Substanz mit emulgierender Wirkung neben der chemisch modifizierten Stärke kleiner als 2 Gew.-% ist, wobei die Gew.-%-Angaben sich jeweils auf das Gesamtgewicht der Emulsion beziehen,
gelöst.

In der erfindungsgemäßen, gebrauchsfertigen, stabilen Emulsion beträgt der Gehalt an dem fettlöslichen Vitamin oder an dem Carotinoid mindestens 2 Gew.-%, bevorzugt 3 bis 30 Gew.-%, besonders bevorzugt 3 bis 12 Gew.-%, insbesondere 3 bis 6 Gew.-%, wobei die Gew.-%-Angaben sich auf das Gesamtgewicht der Emulsion beziehen.

In der erfindungsgemäßen, gebrauchsfertigen, stabilen Emulsion ist der Gehalt einer weiteren Substanz mit emulgierender Wirkung neben der chemisch modifizierten Stärke kleiner als 2 Gew.-%, bevorzugt kleiner als 1 Gew.-%. Handelt es sich bei der weiteren Substanz mit emulgierender Wirkung um Ascorbylpalmitat, so ist der Gehalt an Ascorbylpalmitat bevorzugt geringer als 0,5 Gew.-%, besonders bevorzugt geringer als 0,25 Gew.-%, insbesondere geringer als 0,1 Gew.-%, wobei die Gew.-%-Angaben sich auf das Gesamtgewicht der Emulsion beziehen.

Bei dem fettlöslichen Vitamin kann es sich beispielsweise um die Vitamine A, D, E oder K oder um ein Derivat derselben handeln. Bevorzugt enthält die erfindungsgemäße, gebrauchsfertige, stabile Emulsion ein Carotinoid, wie beispielsweise Canthaxanthin, Astaxanthin, Zeaxanthin, Lutein, Lycopin oder β-Carotin. Bevorzugte Carotinoide sind Lycopin oder β-Carotin, insbesondere β-Carotin.
Die erfindungsgemäße, gebrauchsfertige, stabile Emulsion umfasst bevorzugt eine dispergierte Ölphase, die ein Carotinoid, bevorzugt Lycopin oder β-Carotin, insbesondere β-Carotin, gelöst in einem physiologisch verträglichen Öl umfasst.

Als physiologisch verträgliche Öle kommen prinzipiell Öle synthetischer, mineralischer, pflanzlicher oder tierischer Herkunft in Frage. Beispiele sind Sesamöl, Maiskeimöl, Baumwollsaatöl, Sojabohnenöl, Erdnussöl, Ester mittel-kettiger pflanzlicher Fettsäuren, Oleostearin, Paraffinöl, Glycerylstearat, Isopropylmyristat, Diisopropyladipat, 2-Ethylhexansäurecetylstearylester, hydriertes Polyisobuten, Caprylsäure/Caprinsäure-Triglyceride, Palmöl, Palmkernöl, Lanolin und PUFAs (polyunsaturated fatty acids), wie Eicosapentaensäure (EPA), Docosahexaensäure (DHA) und alpha-Linolensäure.

Bevorzugt sind physiologisch verträgliche Öle pflanzlicher oder tierischer Herkunft, die bei 30 °C flüssig sind, wie Sonnenblumenöl, Palmöl, Palmkernöl, Sesamöl, Maiskeimöl, Baumwollsaatöl, Sojabohnenöl, Erdnussöl, Ester mittelkettiger Triglyceride (sogenannte MCT Öle), Fischöle, wie Makrelen-, Sprotten- oder Lachsöl. Besonders bevorzugt sind physiologisch verträgliche Öle pflanzlicher Herkunft, die weitestgehend nur gesättigte Fettsäuren enthalten, wie beispielsweise Palmkernöl oder Kokosöl, insbesondere die daraus gewinnbaren Ester mittelkettiger Triglyceride.

Die erfindungsgemäße, gebrauchsfertige, stabile Emulsion umfasst eine wässrige Phase, die 30 bis 55 Gew.-% Glycerin, bezogen auf das Gesamtgewicht der Emulsion als physiologisch verträglichen Polyalkohol umfasst.

Bevorzugt beträgt in der erfindungsgemäßen, gebrauchsfertigen, stabilen Emulsion der Gehalt an der chemisch modifizierten Stärke von 5 bis 40 Gew.-%, insbesondere 10 bis 25 Gew.-% bezogen auf das Gesamtgewicht der Emulsion.

Unter chemisch modifizierter Stärke sind chemisch und/oder enzymatisch hergestellte Umwandlungsprodukte der Stärke zu verstehen. Dabei kann es sich um Stärkeether, Stärkeester oder Stärkephosphate handeln. Bevorzugte Vertreter aus dieser Gruppe sind Stärkeester, insbesondere Octenyl-Succinat Stärke wie beispielsweise Capsul^{®} (Natriumoctenylsuccinat Stärke) von der Fa. National Starch, Cleargum CO 01 von Roquette oder Purity^{®} Gum 2000 (Natriumoctenylsuccinat Stärke) von der Fa. National Starch, insbesondere eine Natriumoctenylsuccinat Stärke wie Purity^{®} Gum 2000.

Der Gehalt der dispergierten Ölphase in der erfindungsgemäßen, gebrauchsfertigen, stabilen Emulsion beträgt bevorzugt von 10 bis 40 Gew.-%, insbesondere 10 bis 30 Gew.-% bezogen auf das Gesamtgewicht der Emulsion.

Bevorzugt ist eine erfindungsgemäße, gebrauchsfertige, stabile Emulsion, deren dispergierte Ölphase zu mehr als 90 Gew.-%, insbesondere zu mehr als 95 Gew.-% aus einem Carotinoid und einem physiologisch verträglichem Öl besteht.

Zur Erhöhung der Stabilität des Wirkstoffes gegen oxidativen Abbau ist es vorteilhaft, wenn die Emulsion Stabilisatoren wie α-Tocopherol, t-Butylhydroxytoluol, t-Butylhydroxyanisol, Ascorbinsäure oder Ethoxyquin, insbesondere α-Tocopherol enthält.

Besonders bevorzugt ist eine erfindungsgemäße, gebrauchsfertige, stabile Emulsion, wobei die dispergierte Ölphase von 3 bis 6 Gew.-% β-Carotin und von 7 bis 20 Gew.-% eines mittelkettigen Triglycerids enthält, die wässrige Phase von 10 bis 25 Gew.-% Stärkenatrium-Octenyl-Succinat und von 30 bis 55 Gew.-% Glycerin umfasst und der Gehalt an Ascorbylpalmitat in der Emulsion geringer als 0,5 Gew.-%, bevorzugt geringer als 0,25 Gew.-%, insbesondere geringer als 0,1 Gew.-% ist, wobei sich die Gew.-% Angaben jeweils auf das Gesamtgewicht der Emulsion beziehen.

In der erfindungsgemäßen, gebrauchsfertigen, stabilen Emulsion liegt die dispergierte Ölphase in Form von kleinen Öltröpfchen in der kontinuierlichen wässrigen Phase vor. Im Allgemeinen kann die Teilchengröße der Tröpfchen der dispergierten Ölphase von 100 nm bis 100 µm reichen. Bevorzugt weisen die Tröpfchen der dispergierten Ölphase eine mittlere Teilchengröße von 250 bis 500 nm auf.

Die Angabe der mittleren Teilchengröße bezieht sich auf den volumengewichteten mittleren Durchmesser (siehe Handbuch zu Malvern Mastersizer S, Malvern Instruments Ltd., UK), der mittels Fraunhofer Beugung bestimmt werden kann.

Neben der chemisch modifizierten Stärke können die erfindungsgemäßen gebrauchsfertigen, stabilen Emulsionen weitere Schutzkolloide enthalten. Dafür kommen beispielsweise die folgenden Stoffe in Frage:
Rinder-, Schweine- oder Fischgelatine, insbesondere sauer oder basisch abgebaute Gelatine mit Bloom-Zahlen im Bereich von 0 bis 250, ganz besonders bevorzugt Gelatine A 100, A 200, A 240, B 100 und B 200 sowie niedermolekulare, enzymatisch abgebaute Gelatinetypen mit der Bloom-Zahl 0 und Molekulargewichten von 15.000 bis 25.000 D wie zum Beispiel Collagel A und Gelitasol P (Firma Stoess, Eberbach) sowie Mischungen dieser Gelatine-Sorten.

Stärke, Dextrin, Pektin, Gummi arabicum (Gum acacia), Ligninsulfonate, Chitosan, Polystyrolsulfonat, Alginate, Casein, Caseinat, Methylcellulose, Carboxymethylcellulose, Hydroxypropylcellulose oder Mischungen dieser Schutzkolloide.

Pflanzenproteine wie Soja-, Reis- und/oder Weizenproteine, wobei diese Pflanzenproteine teilabgebaut oder in nicht abgebauter Form vorliegen können.

Die erfindungsgemäße, gebrauchsfertige, stabile Emulsion zeichnet sich unter anderem dadurch aus, dass sie selbst eine gute Lagerstabilität aufweist. Weiterhin lässt sich die erfindungsgemäße Emulsion in Calcium- oder Magnesium-haltigen Sportlergetränken oder in Getränken, die Trinkwasser mit einem hohen Gehalt an Calcium- oder Magnesiumionen enthalten, problemlos einarbeiten, wobei die hergestellten Getränke eine gute Stabilität hinsichtlich einer unerwünschten Ringbildung aufweisen.

Die erfindungsgemäße, gebrauchsfertige, stabile Emulsion eignet sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich. Bevorzugt eignet sich die gebrauchsfertige, stabile Emulsion als Zusatz zu Getränken.

Ein weiter Gegenstand der vorliegenden Erfindung ist daher auch die Verwendung der oben beschriebenen, erfindungsgemäßen gebrauchsfertigen, stabilen Emulsion als Zusatz zu Tierfuttermitteln, Lebensmitteln, Nahrungsergänzungsmitteln sowie kosmetischen und pharmazeutischen Mitteln, insbesondere als Zusatz zu Getränken.

Ebenfalls Gegenstand der vorliegenden Erfindung sind Tierfuttermittel, Lebensmittel und Nahrungsergänzungsmittel, insbesondere ein Getränk, welches die erfindungsgemäße, gebrauchsfertige, stabile Emulsion enthält.

Die Erfindung wird durch folgende, die Erfindung jedoch nicht einschränkende Beispiele erläutert:

### Beispiele

### Beispiel 1

In einem 1 L Becherglas wurden 145 g Wasser und 200 g Glycerin gemischt und auf 60°C erwärmt. Zu dieser Mischung wurden 93 g modifizierte Stärke (Purity Gum 2000 von National Starch) gegeben. Die Mischung wurde bei 60°C für 60 Minuten quellen gelassen.
In einem 250 ml Dreihalskolben wurden 65,5 g β-Carotin Dispersion (Lucarotin 33 MCT, BASF, 33 Gew.-% β-Carotin) in einem auf 180°C erwärmten Ölbad erhitzt, bis das β-Carotin vollständig gelöst war. Die Ölphase wurde dann unter Rühren mit einer Zahnkranzdispergiermaschine (Ultra Turrax) bei 10.000 U/min in die Wasserphase eingeleitet. Nach einer Emulgierzeit von 15 Minuten wurde die Emulsion mittels eines Hochdruckhomogenisators bei 700 bar feinemulgiert. Man erhielt eine wasserdispergierbare Emulsion mit 4 Gew.-% β-Carotin. In Wasser dispergiert wiesen die β-Carotin-Öl-Tröpfchen eine mittlere Partikelgröße von 275 nm auf.

## Patentansprüche

1. Gebrauchsfertige, stabile Emulsion umfassend
a) eine dispergierte Ölphase, die ein fettlösliches Vitamin oder ein Carotinoid umfasst,
und
b) eine wässrige Phase, die 30 bis 55 Gew.-% Glycerin und eine chemisch modifizierte Stärke als wasserlösliches Schutzkolloid umfasst,
worin der Gehalt an dem fettlöslichen Vitamin oder an dem Carotinoid mindestens 2 Gew.-% beträgt, und der Gehalt einer weiteren Substanz mit emulgierender Wirkung, neben der chemisch modifizierten Stärke kleiner als 2 Gew.-% ist, wobei die Gew.-%-Angaben sich jeweils auf das Gesamtgewicht der Emulsion beziehen.

2. Gebrauchsfertige, stabile Emulsion nach Anspruch 1, wobei der Gehalt an dem fettlöslichen Vitamin oder dem Carotinoid von 3 bis 30 Gew.-% bezogen auf das Gesamtgewicht der Emulsion beträgt.

3. Gebrauchsfertige, stabile Emulsion nach Anspruch 1 oder 2, wobei die dispergierte Ölphase ein Carotinoid gelöst in einem physiologisch verträglichen Öl umfasst.

4. Gebrauchsfertige, stabile Emulsion nach einem der Ansprüche 1 bis 3, wobei der Gehalt an der chemisch modifizierten Stärke von 5 bis 40 Gew.-% bezogen auf das Gesamtgewicht der Emulsion beträgt.

5. Gebrauchsfertige, stabile Emulsion nach einem der Ansprüche 1 bis 4, wobei der Gehalt der dispergierten Ölphase von 10 bis 40 Gew.-% bezogen auf das Gesamtgewicht der Emulsion beträgt.

6. Gebrauchsfertige, stabile Emulsion nach einem der Ansprüche 1 bis 5, wobei die dispergierte Ölphase zu mehr als 90 Gew.-% aus einem Carotinoid und einem physiologisch verträglichen Öl besteht.

7. Gebrauchsfertige, stabile Emulsion nach Anspruch 1, wobei die dispergierte Ölphase von 3 bis 6 Gew.-% β-Carotin und von 7 bis 20 Gew.-% eines mittelkettigen Triglycerids enthält, die wässrige Phase von 10 bis 25 Gew.-% Stärkenatrium-Octenyl-Succinat und von 30 bis 55 Gew.-% Glycerin umfasst und der Gehalt an Ascorbylpalmitat geringer als 0,5 Gew-% ist, wobei sich die Gew.-% Angaben jeweils auf das Gesamtgewicht der Emulsion beziehen.

8. Gebrauchsfertige, stabile Emulsion nach einem der Ansprüche 1 bis 7, wobei die Tröpfchen der dispergierten Ölphase eine mittlere Teilchengröße von 250 bis 500nm aufweisen.

9. Verwendung der gebrauchsfertigen, stabilen Emulsion nach einem der Ansprüche 1 bis 8 als Zusatz zu Tierfuttermitteln, Lebensmitteln und Nahrungsergänzungsmitteln sowie kosmetischen und pharmazeutischen Mitteln.

10. Verwendung der gebrauchsfertigen, stabilen Emulsion nach einem der Ansprüche 1 bis 8 als Zusatz zu Getränken.

11. Tierfuttermittel, Lebensmittel, Nahrungsergänzungsmittel, umfassend die gebrauchsfertige, stabile Emulsion gemäß einem der Ansprüche 1 bis 8.

12. Getränk, umfassend die gebrauchsfertige, stabile Emulsion gemäß einem der Ansprüche 1 bis 8.

## Claims

1. A stable emulsion ready for use, comprising
a) a dispersed oil phase which comprises a fat-soluble vitamin or a carotenoid,
and
b) an aqueous phase which comprises 30 to 55% by weight of glycerol and a chemically modified starch as water-soluble protective colloid,
in which the content of the fat-soluble vitamin or of the carotenoid is at least 2% by weight, and the content of a further substance with an emulsifying effect besides the chemically modified starch is less than 2% by weight, where the % by weight data are in each case based on the total weight of the emulsion.

2. The stable emulsion ready for use according to claim 1, where the content of the fat-soluble vitamin or carotenoid is from 3 to 30% by weight based on the total weight of the emulsion.

3. The stable emulsion ready for use according to claim 1 or 2, where the dispersed oil phase comprises a carotenoid dissolved in a physiologically tolerated oil.

4. The stable emulsion ready for use according to any of claims 1 to 3, where the content of the chemically modified starch is from 5 to 40% by weight based on the total weight of the emulsion.

5. The stable emulsion ready for use according to any of claims 1 to 4, where the content of the dispersed oil phase is from 10 to 40% by weight based on the total weight of the emulsion.

6. The stable emulsion ready for use according to any of claims 1 to 5, where the dispersed oil phase consists to an extent of more than 90% by weight of a carotenoid and a physiologically tolerated oil.

7. The stable emulsion ready for use according to claim 1, where the dispersed oil phase comprises from 3 to 6% by weight of β-carotene and from 7 to 20% by weight of a medium-chain triglyceride, the aqueous phase comprises from 10 to 25% by weight of starch sodium octenyl succinate and from 30 to 55% by weight of glycerol, and the ascorbyl palmitate content is less than 0.5% by weight, where the % by weight data are based in each case on the total weight of the emulsion.

8. The stable emulsion ready for use according to any of claims 1 to 7, where the droplets of the dispersed oil phase have an average particle size of from 250 to 500 nm.

9. The use of the stable emulsion ready for use according to any of claims 1 to 8 as addition to animal feeds, human foods and dietary supplements, and cosmetic and pharmaceutical compositions.

10. The use of the stable emulsion ready for use according to any of claims 1 to 8 as addition to beverages.

11. An animal feed, human food or dietary supplement comprising the stable emulsion ready for use according to any of claims 1 to 8.

12. A beverage comprising the stable emulsion ready for use according to any of claims 1 to 8.

## Revendications

1. Émulsion stable prête à l'emploi, comprenant
a) une phase huileuse dispersée, qui comprend une vitamine soluble dans les matières grasses ou un caroténoïde,
et
b) une phase aqueuse, qui comprend 30 à 55 % en poids d'une glycérine et d'un amidon modifié chimiquement en tant que colloïde protecteur soluble dans l'eau,
dans laquelle la teneur en vitamine soluble dans les matières grasses ou en caroténoïde est d'au moins 2 % en poids, et la teneur en une autre substance à effet émulsifiant outre l'amidon modifié chimiquement est inférieure à 2 % en poids, les données de % en poids se rapportant à chaque fois au poids total de l'émulsion.

2. Émulsion stable prête à l'emploi selon la revendication 1, dans laquelle la teneur en vitamine soluble dans les matières grasses ou en caroténoïde est de 3 à 30 % en poids, par rapport au poids total de l'émulsion.

3. Émulsion stable prête à l'emploi selon la revendication 1 ou 2, dans laquelle la phase huileuse dispersée comprend un caroténoïde dissous dans une huile physiologiquement compatible.

4. Émulsion stable prête à l'emploi selon l'une quelconque des revendications 1 à 3, dans laquelle la teneur en amidon modifié chimiquement est de 5 à 40 % en poids, par rapport au poids total de l'émulsion.

5. Émulsion stable prête à l'emploi selon l'une quelconque des revendications 1 à 4, dans laquelle la teneur en phase huileuse dispersée est de 10 à 40 % en poids, par rapport au poids total de l'émulsion.

6. Émulsion stable prête à l'emploi selon l'une quelconque des revendications 1 à 5, dans laquelle la phase huileuse dispersée est constituée à plus de 90 % en poids d'un caroténoïde et d'une huile physiologiquement compatible.

7. Émulsion stable prête à l'emploi selon la revendication 1, dans laquelle la phase huileuse dispersée contient 3 à 6 % en poids de β-carotène et 7 à 20 % en poids d'un triglycéride à chaîne moyenne, la phase aqueuse comprend 10 à 25 % en poids de succinate octénylique d'amidon sodique et de 30 à 55 % en poids de glycérine, et la teneur en palmitate d'ascorbyle est inférieure à 0,5 % en poids, les données de % en poids se rapportant à chaque fois au poids total de l'émulsion.

8. Émulsion stable prête à l'emploi selon l'une quelconque des revendications 1 à 7, dans laquelle les gouttelettes de la phase huileuse dispersée présentent une taille de particule moyenne de 250 à 500 nm.

9. Utilisation de l'émulsion stable prête à l'emploi selon l'une quelconque des revendications 1 à 8 en tant qu'additif pour des aliments pour animaux, des produits alimentaires et des compléments alimentaires, ainsi que des produits cosmétiques et pharmaceutiques.

10. Utilisation de l'émulsion stable prête à l'emploi selon l'une quelconque des revendications 1 à 8 en tant qu'additif pour des boissons.

11. Aliment pour animaux, produit alimentaire et complément alimentaire, comprenant l'émulsion stable prête à l'emploi selon l'une quelconque des revendications 1 à 8.

12. Boisson, comprenant l'émulsion stable prête à l'emploi selon l'une quelconque des revendications 1 à 8 .
